# EUROPEAN PATENT APPLICATION

(11) **EP 3 795 139 A1**
(43) Date of publication of application: **24.03.2021**
(21) Application number: 19198870.8
(22) Date of filing: 23.09.2019
(51) Int. Cl.: A61K 9/00, A61K 9/10, A61K 9/19, A61K 31/00, A61K 47/10, A61K 47/26

(54) **COMPOSITION WITH DRUG MICRO-NANO PARTICLES OF AN ANTI-CANCER AGENT**

(71) Applicant: CAPNOMED GmbH, 78658 Zimmern (DE)
(72) Inventor: Sahoo, Ranjita, 47574 Goch (DE)
(74) Representative: Eisenführ Speiser

(57) **Abstract**

The present invention relates to a composition comprising a water-insoluble anti-cancer agent present as drop particles in water, a method for producing such a composition, a lyophilizate of such a composition and such a composition for use in the treatment of cancer.

## Description

The present invention relates to a composition comprising a water-insoluble anti-cancer agent present as drop particles in water, a method for producing such a composition, a lyophilisate of such a composition and such a composition for use in the treatment of cancer.

Cancer is the second leading cause of death globally, and is responsible for an estimated 9.6 million deaths in 2018. Globally, about 1 in 6 deaths is due to cancer. Among the most common cancers are lung cancer (2.09 million cases, 1.76 million deaths in 2018), breast cancer (2.09 million cases, 627,000 deaths in 2018), colorectal cancer (1.80 million cases, 862,000 deaths in 2018), prostate cancer (1.28 million cases) and stomach cancer (1.03 million cases, 783,000 deaths in 2018).

Metastases are new pathological sites spread by a cancerous (e.g. malignant) tumour. Malignant tumours are capable to invade into adjacent and distant tissue, e.g. by invasion into the blood circulation, followed by invasion into another site, where another tumour (i.e. a metastasis) may grow. Likewise, such tumours may spread along various tissues, which complicates especially a surgical removal of the tumour.

The cancer site as used herein is the part or cavity of the body, i.e. the respective tissue or part of a tissue, where cancer cells located. Cavities also include hollow organs. Often, hollow organs such as the oesophagus, the stomach, the urinary bladder or other organs e.g. containing a virtual space and lined with an epithelium may serve as cancer site.

Particularly in view of cancer sites, the abdominal cavity and the pleural cavities are important regions of the body. The abdomen stretches from the thorax at the thoracic diaphragm to the pelvis at the pelvic brim. The region occupied by the abdomen is termed the abdominal cavity and contains many organs such as the stomach, the small intestine and the colon, the liver, the gall bladder and the pancreas. The abdominal cavity and the pleural cavities are coated by an extensive membrane, called the peritoneum or, respectively, the pleura, which covers the abdominal wall and the pelvic walls (parietal peritoneum) as well as the included organs (visceral peritoneum) or, respectively the inner surface of the thoracic cavity (parietal pleura) as well as the included organs (visceral pleura).

A special form of metastases are abdominal, particularly peritoneal metastases. As the cancer cells are attached to the outside of several organs and tissues but reach into the area of the peritoneum (and are thus classified as abdominal or particularly peritoneal or, respectively, pleural), these metastases are more difficult to reach with medication via the blood circulation. Cancer cells may therefore "escape" into the peritoneum and/or peritoneal cavity. Still, in many cases, these cancer sites are covered by the peritoneum, i.e. are positioned between the respective organ or tissue and the peritoneum or the pleura.

The terms "peritoneal metastasis" and "peritoneal cancer" as used herein may be understood as synonyms. Both terms comprise cancer cells of a primary or a secondary site of the host's body.

The terms "pleural metastasis" and "pleural cancer" as used herein may be understood as synonyms. Both terms comprise cancer cells of a primary or a secondary site of the host's body.

The treatment options for cancer strongly depend on several factors such as the involved organs, the cause of cancer and further complicating factors as well as the age and health status of the patient.

Although many limitations, for various cancer types, a systemic chemotherapy is applied as a standard of care. However, several tissues have a poor vascularisation and can thus be poorly reached by a systemically applied chemotherapy. The therapy thus results in a poor performance. As a counteractive measure, higher doses and/or higher volumes of the anti-cancer agent(s) could be applied transport higher amounts of the anti-cancer agent into the poorly vascularised tissues. However, increasing concentrations also increase the risk of dangerous adverse effects, renal toxicity, neurotoxicity or cardiac toxicity.

As an improvement to systemic chemotherapy, local chemotherapy can be applied to patients with tumour sites in poorly vascularized tissues. The delivery of the chemotherapeutically active substance is thereby localised to a desired tissue or region to achieve desired chemotherapeutic effect.

Such treatment options have recently emerged particularly for cancer sites in the poorly vascularized peritoneal cavity or pleural cavities:
Possible treatment options for peritoneal metastasis are e.g. Hyperthermic Intraperitoneal Chemotherapy (HIPEC) or Pressurized Intraperitoneal Aerosol Chemotherapy (PIPAC). Possible treatment options for pleural metastasis is Pressurized Intrathoracic Aerosol Chemotherapy (PITAC). In some cases, electro-precipitation may also be an option (as described for example in Kakchekeeva et al., In Vivo Feasibility of Electrostatic Precipitation as an Adjunct to Pressurized Intraperitoneal Aerosol Chemotherapy (ePIPAC), Ann Surg Oncol. 2016 Dec;23(Suppl 5):592-598 or in Reymond et al., Electrostatic precipitation Pressurized IntraPeritoneal Aerosol Chemotherapy (ePIPAC): first in-human application, Pleura Peritoneum, 2016 Jun 1;1(2):109-116).

Hyperthermic Intraperitoneal Chemotherapy (HIPEC) is a treatment usually performed after surgery when all visible cancer sites in the abdomen are surgically removed. A liquid heated chemotherapy is applied to the peritoneal cavity, bathing all reachable organs and their surfaces. With this treatment, any remaining cancer cells shall be killed. However, this treatment is a time-consuming process with higher mortality rates.

Pressurized Intraperitoneal Aerosol Chemotherapy (PIPAC) as well as Pressurized Intrathoracic Aerosol Chemotherapy (PITAC) are palliative treatments of peritoneal or, respectively, pleural metastasis. PIPAC is a method for applying chemotherapeutic drugs, in form of an aerosol, under pressure into the abdominal cavity. The aerosol is usually applied within the closed abdominal or, respectively, thoracic cavity, i.e. during a laparoscopy or, respectively, thoracoscopy. PIPAC and PITAC are significantly time saving therapies compared to other available techniques like HIPEC.

In many cases, however, sole chemotherapy is not sufficient for treating cancer and a surgery, in which the cancer cells are removed, becomes necessary or even the only remaining option. Surgeries generally provide risks to patients such as (internal) bleedings, infections and stress for the cardiovascular system during anaesthesia. Nevertheless, surgeries are usually necessary if performed. However, to prevent the risks involved, unnecessary surgeries should be avoided.

A drawback of the surgical removal of cancer cells is that in several cases, some cancer cells or cancer cell clusters remain in the body, as they were too small to be seen or detected during surgery. These cancer cells or cancer cells clusters usually tend to be very aggressive, i.e. are rapidly growing and likely to spread in form of metastases.

Furthermore, healing includes the rapid growth of tissue in proximity of the wound, which is usually triggered or accompanied by a mixture of endogenous growth stimulants. Such stimulants may also promote growth of the remaining cancer cells or cancer cell clusters.

As the body is weakened by performing the surgery, a systemic chemotherapy to kill the remaining cancer cells or cancer cell clusters is not an option. Also, local chemotherapy often cannot be performed, as the substances themselves usually interfere with the healing process or as the local application of the anti-cancer agent is performed under pressure (e.g. in case of PIPAC or PITAC) or other (physical) interactions with the tissue affected by surgery.

Moreover, even if the anti-cancer agents reach their destination, their concentration must be low enough to ensure proper wound healing but at the same time high enough to fight the remaining cancer cells or cancer cell clusters. Thus, their concentration needs to be tightly regulated over time and be analysed to intervene if it becomes necessary.

Furthermore, as any current palliative chemotherapy, PIPAC and PITAC have to be repeated at regular intervals (currently about 6 weeks) to be effective.

On the other side, delivery of chemotherapeutics to the peritoneum at the same time as surgery is performed, might prevent tumour recurrence, however, is often associated with wound healing problems, in particular with bowel perforations and leakage of surgical sutures.

Therefore, it was the primary object of the present invention to provide new and improved treatment measures for treating cancer or in connection with treating cancer, particularly shortly after a surgery for removing a tumour, preferably to provide new and improved delivery systems to be used in the treatment of cancer, wherein one or more, particularly preferably all, of the above-mentioned drawbacks are avoided or overcome by use of the same.

The primary object of the present invention is achieved by a composition comprising or consisting of
a) one, two, three or more anti-cancer agent(s),
   wherein the or all anti-cancer agent(s) is/are water-insoluble,
b) optionally one, two, three or more immunomodulatory agent(s),
c) one, two, three or more surfactant(s),
d) optionally one, two, three or more solvent(s),
e) optionally one, two, three or more coating material(s),
f) water,
g) and optionally one or more further pharmaceutically acceptable components,
   wherein the or all anti-cancer agent(s) form drop particles in component f) of the composition,
   wherein the particle size of at least 80 %, preferably at least 85 %, particularly preferably at least 90 % of the drop particles is ≤ 500 nm,
   wherein the ratio of the total weight of component a) to the total weight of component c) is in a range of from 1:20 to 20:1, preferably of from 1:10 to 10:1, particularly preferably of from 1:8 to 7.5:1.

The term "water-insoluble" substances describes substances which are not or which are only poorly soluble in water.

The term "drop particle" as used herein is preferably meant to be a particle of an anti-cancer agent and/or a mixture of anti-cancer agents. The particles may have any shape, e.g. spherical, triangular, crystal, square, hexagonal or any possible shape, be present in form of small needles or be present as any combination thereof. Advantageously the surfactant(s) in the composition stabilize the drop particles and prevent the particle aggregation.

The term "the or all anti-cancer agent(s) form drop particles in component f) of the composition" is meant to be understood broadly, i.e. for example such that the or all anti-cancer agent(s) form drop particles in water or, respectively in an aqueous phase of the composition.

The term "a particle of an anti-cancer agent and/or a mixture of anti-cancer agents" is meant to be understood such that the composition comprises particles with one, two, three or more anti-cancer agent(s), wherein in case several anti-cancer agent(s) are present in the composition, these may be present in separate particles each and/or as any mixture in the same particles (i.e. the mixture may be the same in all particles or may also vary between the particles).

Thus, the particles in the composition may preferably include
a) only one anti-cancer agent,
b) two, three or more anti-cancer agents, wherein all of said particles include only one of the anti-cancer agents,
c) two, three or more anti-cancer agents, wherein all of said particles include a mixture of the anti-cancer agents, which is the same, with regard to the selection of the anti-cancer agents, in all particles,
d) two, three or more anti-cancer agents, wherein all of said particles include a mixture of the anti-cancer agents, which may be different between the particles,
e) two, three or more anti-cancer agents, wherein some of said particles include only one of the anti-cancer agents and some of said particles include a mixture of the anti-cancer agents, which is the same, with regard to the selection of the anti-cancer agents, in all particles including a mixture of anti-cancer agents,
f) two, three or more anti-cancer agents, wherein some of said particles include only one of the anti-cancer agents and some of said particles include a mixture of the anti-cancer agents, which may be different between the particles including a mixture of anti-cancer agents,
   or
g) any possible combination thereof.

The composition according to the invention preferably comprises or consists of
a) 1 to 30 wt.-%, based on the total weight of the composition, of one, two, three or more anti-cancer agent(s),
b) 1 to 10 wt.-%, based on the total weight of the composition, of one, two, three or more immunomodulatory agent(s),
c) 4 to 8 wt.-%, based on the total weight of the composition, of one, two, three or more surfactant(s),
d) optionally: 5 to 20 wt.-%, based on the total weight of the composition, of one, two, three or more solvent(s),
e) optionally: 0.1 to 0.25 wt.-%, based on the total weight of the composition, of one, two, three or more coating material(s),
f) water,
g) and optionally one or more further pharmaceutically acceptable components.

Preferably, component b) is present and the ratio of the total weight of component a) to the total weight of component b) is in a range of from 1:20 to 50:1, preferably of from 1:15 to 40:1, particularly preferably of from 1:10 to 30:1.

Additionally or alternatively, component b) is present and the ratio of the total weight of component b) to the total weight of component c) is in a range of from 1:25 to 20:1, preferably of from 1:20 to 10:1, particularly preferably of from 1:10 to 5:1, especially preferably of from 1:8 to 2.5:1.

Preferably, component d) is present and the ratio of the total weight of component a) to the total weight of component d) is in a range of from 1:50 to 20:1, preferably of from 1:25 to 10:1, particularly preferably of from 1:20 to 6:1.

Additionally or alternatively, component d) is present and the ratio of the total weight of component c) to the total weight of component d) is in a range of from 1:20 to 10:1, preferably of from 1:20 to 5:1, particularly preferably of from 1:10 to 2:1, especially preferably of from 1:5 to 1.6:1.

Preferably, component e) is present and the ratio of the total weight of component a) to the total weight of component e) is in a range of from 0.5:1 to 500:1, preferably of from 1:1 to 350:1, particularly preferably of from 4:1 to 300:1.

Additionally or alternatively, component e) is present and the ratio of the total weight of component c) to the total weight of component e) is in a range of from 5:1 to 150:1, preferably of from 10:1 to 100:1, particularly preferably of from 16:1 to 80:1.

Preferably, component b) and component e) are present and the ratio of the total weight of component b) to the total weight of component e) is in a range of from 1:1 to 200:1, preferably of from 2:1 to 150:1, particularly preferably of from 4:1 to 100:1.

It is also preferred that the particle size of at least 30 %, preferably at least 40 %, particularly preferably at least 45 %, especially preferably 50 % of the drop particles is ≥ 250 nm and ≤ 300 nm.

Additionally or alternatively, it is also preferred that at least 15 % of the particles, preferably at least 20 %, at least 25 %, at least 30 %, at least 35 %, at least 40 %, at least 45 %, at least 50 %, at least 55 %, at least 60 %, at least 65 %, at least 70 %, or at least 75 % have a particle size of from 140 to ≤ 250 nm.

Additionally or alternatively, it is also preferred that at least 15 % of the particles, preferably at least 20 %, at least 25 %, at least 30 %, at least 35 %, at least 40 %, at least 45 %, at least 50 %, at least 55 %, at least 60 %, at least 65 %, at least 70 %, or at least 75 % have a particle size of from > 250 to 500 nm.

Preferably, at least 75 %, at least 76 %, at least 77 %, at least 78 %, at least 79 %, at least 80 %, at least 81 %, at least 82 %, at least 83 %, at least 84 %, at least 85 %, at least 86 %, at least 87 %, at least 88 %, at least 89 %, at least 90 %, at least 91 %, at least 92 %, at least 93 %, at least 94 %, at least 95 %, at least 96 %, at least 97 %, at least 98 %, at least 99 %, or all of the particles in the delivery system have a particle size in a range of from 140 to 500 nm.

It is further preferred that the average particle size of the total of the particles in the active substance delivery system is in a range of from 50 to 1000 nm, preferably of from 100 to 750 nm, particularly preferably of from 140 to 500 nm, especially preferably of from 200 to 550 nm, further preferably of from 250 to 500 nm. It is also preferred that the average particle size of the total of the particles in the active substance delivery system is in a range of from 150 to 300 nm, preferably of from 175 to 250 nm. It is also preferred that the average particle size of the total of the particles in the active substance delivery system is in a range of from 350 to 550 nm, preferably of from 400 to 500 nm.

Typically, the particle size of the particles is measured by dynamic light scattering techniques and electron microscopy or scanning probe microscopy. The size can also be determined by fractionation with disc centrifugation and flow techniques. Further methods are known to a person skilled in the art and may also be applied. It is preferred that the (average) particle size as described herein is defined by applying such methods. Particularly, the (average) particle size as described herein is defined by applying dynamic light scattering techniques, laser diffraction, electron microscopy, scanning probe microscopy, fractionation with disc centrifugation, flow techniques and/or other methods known to a person skilled in the art. Preferably, the (average) particle size describes the average of the particle sizes of a plurality of particles determined as described above. Furthermore, when measuring the (average) particle size, preferably the mean diameter of the respective particle(s) is considered.

The particular particle sizes may be achieved by methods as described below. Furthermore, it is of particular advantage to use one or more surfactants in the composition, as these will stabilize the respective particles and prevent their aggregation. Thus, the one or more surfactants stabilize the (average) particle size.

The term "anti-cancer agent" preferably describes approved anti-cancer agents as well as any other medication or substance exhibiting anti-cancer activity, e.g. although typically used for other purposes.

The term "anti-cancer activity" preferably includes a reduction in tumour size, tumour growth, tumour cell number, tumour motility and/or tumour migration.

Preferably, the, one, two, three or all anti-cancer agent(s) is/are selected from the group consisting of anti-cancer agents used against a cancer type selected from the group consisting of gastrointestinal cancer, particularly gastric cancer, colorectal cancer, hepatobiliary or pancreatic cancer, appendix cancer, esophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin.

Examples of anti-cancer agents which may be used according to the invention are cisplatin, doxorubicin, paclitaxel, oxaliplatin, clofazimine and quercetin.

Preferably, the, one, two, three or more or all immunomodulatory agent(s) of component b), if present, is/are selected from the group consisting of curcumin, lutein, flavonoids like hesperidin, apigenin, hesperetin, aioene, arctigenin, β-carotene, Lycopene epigallocate-chin-3-gallate, ginsan, glabridin and guinic acid.

The composition according to the invention may advantageously contain one or more immunomodulatory agent(s) which may prevent or alleviate possible side-effects of the, one, two, three or more or all anti-cancer agent(s) also present in the composition, thus fastening the healing process of a patient. The particular immunomodulatory agent(s) may be selected with regard of the present anti-cancer agent(s).

Also preferably, the, one, two, three or more or all surfactant(s) of component c) is/are selected from the group consisting of poloxamer, such as poloxamer 188, polysorbate, such as polysorbate 80, phosphatidylcholine, sorbitan fatty acid esters, such as span 80, vitamin E derivatives, such as D-α-Tocopheryl polyethylene glycol 1000 succinate (TPGS), polyvinyl alcohols, Macrogol glycerol ricinoleate, Polyoxyl-35-castor oil, Span, Macrogol 15 Hydroxystearate, Polyoxyl 15 Hydroxystearate and Polyethoxylated 12-hydroxystearic acid.

It is further preferred that the, one, two, three or more or all solvent(s) of component d), if present, is/are solvents that are pharmaceutically acceptable. Preferably, the solvents comprise or consist of one or more substances selected from the group consisting of ethanol, water, acetone, ethyl acetate, chlorinated solvents, DMF, DMSO, (diluted) acetic acid, methylated solvents, tetrahydrofuran, halogenated hydrocarbons such as chloroform and dichloromethane, dioxanes, acetonitrile, polyethylene glycol, such as polyethylene glycol 400; N-methylpyrrolidone; dimethylacetamide; propylene glycol and combinations thereof.

Also preferably, the, one, two, three or more or all coating material(s) is/are selected from the group consisting of celluloses, such as methyl cellulose, hydroxypropyl cellulose, sodium carboxymethyl cellulose or hydroxypropylmethyl cellulose, chitosan or carboxymethyl cellulose, polyvinyl alcohol, polyacrylic acid, polyvinylpyrrolidone, sodium alginate, pectin, carbomer, tragacanth, acacia, gelatin, starch, such as gellatinised starch, gums such as gellan gum, xanthum gum, locust bean gum or acacia gum, hydrogels, folic acid, antibodies, dextran, and serum albumin.

It is also preferred that component e) is present in the composition and at least 50 %, preferably at least 60 %, preferably at least 70 %, preferably at least 80 %, preferably at least 90 %, preferably at least 95 % or substantially all of the drop particles are coated with the or at least one, two, three or more or all of the coating material(s) of component e).

The term "substantially all" of the particles of the composition are coated is meant to be understood such that all particles of the composition except for such particles which were destroyed, damaged and/or otherwise negatively affected before, during and/or after any process acting on the particles are necessarily coated. Typically, the term "substantially all" of the particles of the delivery system are coated is meant to be understood such that at least 96 %, at least 97 %, at least 98 %, at least 99 % are coated.

It is of particular advantage to coat particles with one, two, three or more targeting ligands as in this way, the ligands bind to a targeted structure and the particles are kept in close distance to the targeted structure or are directly attached to it. The release of the anti-cancer agent(s) and optionally immunomodulatory drug(s) is thus performed directly nearby the targeted structure which is preferably the structure to be treated, e.g. the cancer cells to be treated. In this way, the amount of administered particles, and thus also the total amount of anti-cancer agent(s) and optionally immunomodulatory drug(s) applied, may be increased as the effect of said substances is directed, preferably substantially restricted to the targeted structure, whereas an effect on other structures, i.e. preferably non-cancer cells, is reduced or preferably substantially prevented.

Preferably, the, one or all further pharmaceutically acceptable components is/are selected from the group consisting of carriers, polymers, stabilizers, wetting agents, emulsifiers, antioxidants, pH influencing agents, disintegrants, recrystallization agents, fluxing agents, preservatives, solvents, salts, fillers, binders, foamers, defoamers, lubricants, adsorbents, agents for adjusting the osmotic pressure and buffers. In case a pharmaceutically acceptable component contains water, said water is considered to belong to component f) for calculating any weight ratios or wt.-% indications.

It is of particular advantage if the drop particles in the composition have a small particle size. In this case, the particles, i.e. the anti-cancer agent(s) contained therein, may penetrate the tissue more easily, more quickly and/or more deeply. Thus, in total a higher amount of the anti-cancer agent (s) may be applied to the patient, particularly by local application, as the applied small particles of the delivery system quickly penetrate the targeted tissue and do not spread to further tissues which are not intended to be contacted with the active substance. This may be of particular importance for applying anti-cancer agents in higher amounts.

The provision of the anti-cancer agent(s) to the targeted structure depends on the water-solubility of the respective anti-cancer agent(s) as well as on the amount of surfactants in the composition.

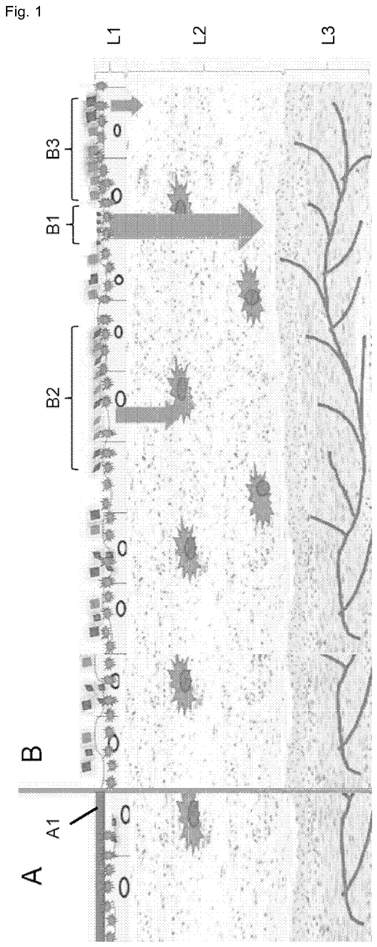

As also shown in Fig.1, smaller particles penetrate the targeted structure more quickly than larger particles. Also, smaller particles dissolve faster and thus release the anti-cancer agent(s) and optionally the immunomodulatory drug(s) more quickly. Medium sized particles show a comparably slower penetration and dissolve at a moderate rate compared to smaller particles. However, medium size particles prevent a slower but steady / prolonged release. Moreover, larger particles dissolve slower and prevent a slower release which is however sustained / prolonged compared to smaller or medium size particles. Furthermore, larger particles penetrate more slowly or may even only penetrate after releasing a certain amount of the anti-cancer agent(s) and optionally the immunomodulatory drug(s) and thus after reducing their size. Therefore, the provision of the anti-cancer agent(s) to the targeted structures may be controlled by balancing the (relative) amounts of smaller, medium and larger particles (as described herein). I.e., applying the anti-cancer agent(s) in smaller, medium and larger particles provides a release over a prolonged time frame.

The present invention further relates to a method for producing a composition according to the invention, comprising or consisting of the steps
i) providing one, two, three or more anti-cancer agent(s), wherein the or all anti-cancer agent(s) is/are water-insoluble,
ii) providing one, two, three or more surfactant(s),
iii) optionally: providing one, two, three or more immunomodulatory agent(s),
iv) optionally: providing one, two, three or more solvent(s),
v) optionally: providing one, two, three or more coating material(s)
vi) optionally: providing one or more further pharmaceutically acceptable components,
vii) providing water,
viii) mixing the components provided in steps i) and ii) and optionally iii) and/or iv) and/or v) and/or vi) with the water provided in step vii), and homogenizing the composition obtained in step viii) and obtaining drop particles by applying
   a) bead milling or
   b) high pressure homogenization or
   c) a microfluidic process or
   d) combinations thereof
ix) optionally: providing one or more further pharmaceutically acceptable components and adding these to the composition obtained in step viii), wherein the addition may be performed at any time within step viii).

The mixing of the provided components in step viii) may be performed with all provided components simultaneously or with distinct components at first and (the) others subsequently. The same applies for the one or more further pharmaceutically acceptable components if present.

In case the, one, two, three or more anti-cancer agent(s) and/or the, one, two, three or more immunomodulatory agent(s) is /are fragile substances such as e.g. an antibody, proteins, folic acid, amino acids etc, the respective anti-cancer agent(s) and/or immunomodulatory agent(s) may also be added during the homogenization in step viii), preferably in one of the last cycles of e.g. high pressure homogenization or bead milling.

The homogenization in step viii) is advantageously performed to reduce the size of the drop particles. Preferably, the homogenization in step viii) is performed by a microfluidic (capillary) process with a flow rate of from e.g. 0.2 to 5 ml/min and at e.g. room temperature. The (average) size of the drop particles depends on the particular flow rate. By adjusting a lower flow rate and thereby increasing an efficient mixing of the two streams, smaller drop particles are obtained. In case a microfluidic (capillary) process is applied, it is preferred that component d) of the composition is present.

Also preferably, the mixing and homogenizing step viii) is performed by high pressure homogenization with for example a sequence of 14 to 26 cycles at increasing pressure, e.g. 2 cycles at 200 bar, 2 cycles at 500 bar, 2 cycles at 1000 bar and subsequently 15-20 cycles at 1500 bar. The (average) size of the drop particles depends on the amount of cycles and the set homogenization pressure. By adjusting a higher amount of cycles or a higher pressure, smaller drop particles are obtained.

Also preferably, the mixing and homogenizing step viii) is performed by bead milling with e.g. a Bühler bead mill and for example zirconia or polystyrene beads, which will be removed by filtration after the milling process. The (average) size of the drop particles depends on the material of the beads, the size of bead and the number of milling cycles. By adjusting a higher amount of cycles, smaller bead size, narrow sized drop particles are obtained.

Also preferably, the mixing and homogenizing step viii) is performed by a combination of the above approaches.

The invention further relates to a composition obtainable or obtained by the method according to the invention. What is generally said herein with regard to the composition also applies to a composition obtainable or obtained by the method according to the invention.

The invention further relates to a lyophilisate of a composition according to the invention, obtainable or obtained by the following step
- lyophilising a composition according to the invention,
   preferably wherein one, two, three or more cryoprotectant(s) is/are used,
   particularly preferably wherein the, one, two, three or more or all cryoprotectant(s) is/are selected from the group consisting of trehalose, DMSO, lactose, Ethylene glycol, sorbitol, glucose, fructose, Glycerol, 2-Methyl-2,4-pentanediol (MPD), Propylene glycol, Sucrose, albumin, mannitol and combination thereof.

It is particularly advantageous for stability, storing and for transporting a composition according to the invention to provide a lyophilisate of said composition. For lyophilisation, common techniques may be applied.

The invention further relates to a composition, as described herein, for use in the treatment of cancer in a human subject, wherein the treatment comprises one or more administration(s) of the composition to the human subject, preferably by direct introduction into the abdomen and/or thorax.

Preferably, the treatment is directed to a cancer type selected from the group consisting of gastro-intestinal cancer, particularly gastric cancer, colorectal cancer, hepatobiliary or pancreatic cancer, appendix cancer, esophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin.

Preferably, the or one, two, three or all administration(s) of the composition according to the invention is/are performed with an assisting tool selected from the group consisting of microneedles, spray devices, angio-injectors, or any combination thereof, preferably with a nebulizer, spraying gun or spray catheter, particularly preferably the assisting tool is a laparoscopic nebulizer, especially preferably the laparoscopic nebulizer known as Capnopen, preferably as described in US 9,511,197 B2.

Preferably, the or one, two, three or all administration(s) comprise or consist of intraperitoneal administration, intravenous injection, oral administration, intramuscular, PIPAC as described above, or combinations thereof or other suitable route of administration known by a skilled person.

In standard drug applications, e.g. in case of PIPAC as described herein, the active substances are typically dissolved in an aqueous carrier e.g. saline and applied to the patient. In this case, the solution forms a thin film on the tissue it is applied on. This film is however fluid, and over the time it accumulates due to gravitational force. At the beginning the active substances to be provided are thus spread over a large area, particularly on areas, i.e. cells, which are not intended to be contacted with the active substances. By applying a composition according to the invention including drop particles as described herein, the anti-cancer agent(s) is/are deposited on the cell surfaces and attach to it. This effect is even enhanced by coating the particles with one, two, three or more targeting ligands as described above. Even in case the tumour is growing and after certain time displacing the applied solution due to its three-dimensional structure (especially in case of a thin film formed by standard drug application), the particles as described herein are typically attached to the tumour cells and are thus not displaced by tumour growth.

Additionally, the application of both, hydrophilic active substances and lipophilic substances, is made possible with the delivery system as described herein.

It is preferred that the treatment comprises one, two, three or more administration(s) of the composition according to the invention, particularly preferably one administration of the composition according to the invention.

The amount of the or, respectively, each anti-cancer agent is preferably contained in a range of from 120 mg to 2500 mg, preferably of from 150 mg to 2000 mg, particularly preferably of from 150 mg to 1500 mg, especially preferably of from 150 mg to 800 mg in the composition according to the invention, preferably this indication refers to the total amount administered during one application.

Additionally or alternatively, the amount of the or, respectively, each anti-cancer agent is preferably contained in a range of from 30 mg/m² body surface to 250 mg/m² body surface, preferably of from 40 mg/m² body surface to 200 mg/m² body surface, particularly preferably of from 50 mg/m² body surface to 180 mg/m² body surface, especially preferably of from 50 mg/m² body surface to 150 mg/m² body surface in the composition according to the invention, wherein this indication refers to the total amount administered during one application, preferably as a powder or suspended or redispersed in physiological solutions like saline. The amounts of the anti-cancer agent may, however, vary with the respective anti-cancer agent(s) and/or type of anti-cancer agent(s). Thus, the amount of the or, respectively, each anti-cancer agent as described may e.g. also be contained in a range of from 50 mg/m² body surface to 70 mg/m² body surface.

The term "body surface" as used herein refers to the body surface of the human subject receiving the delivery system according to the invention or, respectively, the particles according to the invention. The body surface can be calculated from the size of the human subject and its body weight and is well known to a person skilled in the art.

It is also preferred that the treatment with the delivery system as described herein is directed to reducing tumour size and the amount of tumour sites. Tumour sites includes the primary tumour as well as metastases and further tumour reproductions. The treatment can also be directed to prevent or delay cancer recurrence after tumour removal or partial tumour removal, preferably after a surgical tumour removal.

Fig. 1 depicts the mechanism of action of a composition according to the invention (B) versus a standard drug solution (A). The figure shows a cross-section of the peritoneal cavity with the mesothelial layer (L1), the sub-mesothelial zone (L2) and the adipose and connective tissue including blood vessels (L3). For the standard drug solution (A) the formation of a thin film (A1) on the surface of the peritoneal cavity is shown. The composition according to the invention (B) includes smaller particles (B1), medium particles (B2) and larger particles (B3). The penetration of said particles into the tissue is indicated by the arrows below the particles.

Preferred embodiments and further aspects of the present invention also emerge from the attached patent claims and the following examples, wherein the present invention is not limited to these examples.

### Examples:

### Example 1: Mixing and homogenization by microfluidic (capillary) process

| **Ingredient [g]** | **F1** | **F2** | **F3** | **F4** | **F5** | **F6** |
|---|---|---|---|---|---|---|
| Paclitaxel | 1 | 1 | 2 | 1 | 1 | 2 |
| Clofazimine | 1 | 2 | 2 | 1 | 2 | 2 |
| Curcumin | 2 | 2 | 2 | 2 | 2 | 2 |
| Lutein | 0 | 0 | 0 | 0 | 0 | 0 |
| Quercetin | 0 | 0 | 0 | 0 | 0 | 0 |
| Tween 80 | 4 | 4 | 4 | 0 | 0 | 0 |
| Poloxamer | 0 | 0 | 0 | 4 | 4 | 4 |
| DMSO | 10 | 10 | 10 | 10 | 10 | 10 |
| DMF | 10 | 10 | 10 | 10 | 10 | 10 |
| Water | 72 | 71 | 70 | 72 | 71 | 70 |

| **Ingredient [g]** | **F7** | **F8** | **F9** | **F10** | **F11** | **F12** |
|---|---|---|---|---|---|---|
| Paclitaxel | 1 | 1 | 2 | 1 | 1 | 2 |
| Clofazimine | 1 | 2 | 2 | 1 | 2 | 2 |
| Curcumin | 0 | 0 | 0 | 0 | 0 | 0 |
| Lutein | 2 | 2 | 2 | 2 | 2 | 2 |
| Quercetin | 0 | 0 | 0 | 0 | 0 | 0 |
| Tween 80 | 4 | 4 | 4 | 0 | 0 | 0 |
| Poloxamer | 0 | 0 | 0 | 4 | 4 | 4 |
| Ethanol | 20 | 20 | 20 | 20 | 20 | 20 |
| Water | 72 | 71 | 70 | 72 | 71 | 70 |

| **Ingredient [g]** | **F13** | **F14** | **F15** | **F16** | **F17** | **F18** |
|---|---|---|---|---|---|---|
| Paclitaxel | 1 | 1 | 2 | 1 | 1 | 2 |
| Clofazimine | 1 | 2 | 2 | 1 | 2 | 2 |
| Curcumin | 0 | 0 | 0 | 0 | 0 | 0 |
| Lutein | 0 | 0 | 0 | 0 | 0 | 0 |
| Quercetin | 2 | 2 | 2 | 2 | 2 | 2 |
| Tween 80 | 4 | 4 | 4 | 0 | 0 | 0 |
| Poloxamer | 0 | 0 | 0 | 4 | 4 | 4 |
| Ethanol | 20 | 20 | 20 | 20 | 20 | 20 |
| Water | 72 | 71 | 70 | 72 | 71 | 70 |

The above indicated ingredients were provided and subsequently mixed and homogenized by a M-110EH-30 pilot scale machine (Microfluidic corp.) with a flow rate of 1 ml/min at a pressure of 800 bar to obtain the final product.

### Example 2: Mixing and homogenization by high pressure homogenization

| **Ingredient [g]** | **F1** | **F2** | **F3** | **F4** | **F5** | **F6** |
|---|---|---|---|---|---|---|
| Paclitaxel | 1 | 1 | 2 | 1 | 1 | 2 |
| Clofazimine | 1 | 2 | 2 | 1 | 2 | 2 |
| Curcumin | 2 | 2 | 2 | 2 | 2 | 2 |
| Lutein | 0 | 0 | 0 | 0 | 0 | 0 |
| Quercetin | 0 | 0 | 0 | 0 | 0 | 0 |
| Tween 80 | 4 | 4 | 4 | 0 | 0 | 0 |
| Poloxamer | 0 | 0 | 0 | 4 | 4 | 4 |
| Ethanol | 5 | 5 | 5 | 5 | 5 | 5 |
| Water | 87 | 86 | 85 | 87 | 86 | 85 |

| **Ingredient [g]** | **F7** | **F8** | **F9** | **F10** | **F11** | **F12** |
|---|---|---|---|---|---|---|
| Paclitaxel | 1 | 1 | 2 | 1 | 1 | 2 |
| Clofazimine | 1 | 2 | 2 | 1 | 2 | 2 |
| Curcumin | 0 | 0 | 0 | 0 | 0 | 0 |
| Lutein | 2 | 2 | 2 | 2 | 2 | 2 |
| Quercetin | 0 | 0 | 0 | 0 | 0 | 0 |
| Tween 80 | 4 | 4 | 4 | 0 | 0 | 0 |
| Poloxamer | 0 | 0 | 0 | 4 | 4 | 4 |
| Ethanol | 5 | 5 | 5 | 5 | 5 | 5 |
| Water | 87 | 86 | 85 | 87 | 86 | 85 |

| **Ingredient [g]** | **F13** | **F14** | **F15** | **F16** | **F17** | **F18** |
|---|---|---|---|---|---|---|
| Paclitaxel | 1 | 1 | 2 | 1 | 1 | 2 |
| Clofazimine | 1 | 2 | 2 | 1 | 2 | 2 |
| Curcumin | 0 | 0 | 0 | 0 | 0 | 0 |
| Lutein | 0 | 0 | 0 | 0 | 0 | 0 |
| Quercetin | 2 | 2 | 2 | 2 | 2 | 2 |
| Tween 80 | 4 | 4 | 4 | 0 | 0 | 0 |
| Poloxamer | 0 | 0 | 0 | 4 | 4 | 4 |
| Ethanol | 5 | 5 | 5 | 5 | 5 | 5 |
| Water | 87 | 86 | 85 | 87 | 86 | 85 |

The above indicated ingredients were provided and subsequently mixed and homogenized by high pressure homogenization with 2 cycles at 200 bar, 2 cycles at 500 bar, 2 cycles at 1000 bar and subsequently 15 cycles at 1500 bar to obtain the final product.

### Example 3: Grinding by bead milling

| **Ingredient [g]** | **F1** | **F2** | **F3** | **F4** | **F5** | **F6** |
|---|---|---|---|---|---|---|
| Paclitaxel | 1 | 1 | 2 | 1 | 1 | 2 |
| Clofazimine | 1 | 2 | 2 | 1 | 2 | 2 |
| Curcumin | 2 | 2 | 2 | 2 | 2 | 2 |
| Lutein | 0 | 0 | 0 | 0 | 0 | 0 |
| Quercetin | 0 | 0 | 0 | 0 | 0 | 0 |
| Tween 80 | 4 | 4 | 4 | 0 | 0 | 0 |
| Poloxamer | 0 | 0 | 0 | 4 | 4 | 4 |
| Ethanol | 5 | 5 | 5 | 5 | 5 | 5 |
| Water | 87 | 86 | 85 | 87 | 86 | 85 |

| **Ingredient [g]** | **F7** | **F8** | **F9** | **F10** | **F11** | **F12** |
|---|---|---|---|---|---|---|
| Paclitaxel | 1 | 1 | 2 | 1 | 1 | 2 |
| Clofazimine | 1 | 2 | 2 | 1 | 2 | 2 |
| Curcumin | 0 | 0 | 0 | 0 | 0 | 0 |
| Lutein | 2 | 2 | 2 | 2 | 2 | 2 |
| Quercetin | 0 | 0 | 0 | 0 | 0 | 0 |
| Tween 80 | 4 | 4 | 4 | 0 | 0 | 0 |
| Poloxamer | 0 | 0 | 0 | 4 | 4 | 4 |
| Ethanol | 5 | 5 | 5 | 5 | 5 | 5 |
| Water | 87 | 86 | 85 | 87 | 86 | 85 |

| **Ingredient [g]** | **F13** | **F14** | **F15** | **F16** | **F17** | **F18** |
|---|---|---|---|---|---|---|
| Paclitaxel | 1 | 1 | 2 | 1 | 1 | 2 |
| Clofazimine | 1 | 2 | 2 | 1 | 2 | 2 |
| Curcumin | 0 | 0 | 0 | 0 | 0 | 0 |
| Lutein | 0 | 0 | 0 | 0 | 0 | 0 |
| Quercetin | 2 | 2 | 2 | 2 | 2 | 2 |
| Tween 80 | 4 | 4 | 4 | 0 | 0 | 0 |
| Poloxamer | 0 | 0 | 0 | 4 | 4 | 4 |
| Ethanol | 5 | 5 | 5 | 5 | 5 | 5 |
| Water | 87 | 86 | 85 | 87 | 86 | 85 |

The above indicated ingredients were provided and subsequently mixed and milled by bead milling at 6 passes using zirconia beads (0.2 to 0.4mm).

The obtained particles were coated by adding 0.18 g of hydroxypropylmethyl cellulose, in the 3rd stage of passage.

### Example 4: Particle size

The mean particle size of the compositions F1 to F18 of Example 3 was measured and found to be in the following ranges (indicated in nm):

| **F1** | **F2** | **F3** | **F4** | **F5** | **F6** | **F7** | **F8** | **F9** |
|---|---|---|---|---|---|---|---|---|
| 120-250 | 220-270 | 220-310 | 200-240 | 130-250 | 220-280 | 220-280 | 240-280 | 250-350 |

| **F10** | **F11** | **F12** | **F13** | **F14** | **F15** | **F16** | **F17** | **F18** |
|---|---|---|---|---|---|---|---|---|
| 210-260 | 230-250 | 260-320 | 200-260 | 240-290 | 250-320 | 200-250 | 240-290 | 275-350 |

### Example 5: Lyophilisate

Compositions F13,14 to F18 of Example 2 were lyophilised by using mannitol as cryoprotectant. The freeze drying (Alpha 1-2 LDplus) was performed at a temperature of -70°C and vacuum 0.09 mbar for 72 hours.

## Claims

1. Composition comprising or consisting of
a) one, two, three or more anti-cancer agent(s),
wherein the or all anti-cancer agent(s) is/are water-insoluble,
b) optionally one, two, three or more immunomodulatory agent(s),
c) one, two, three or more surfactant(s),
d) optionally one, two, three or more solvent(s),
e) optionally one, two, three or more coating material(s),
f) water,
g) and optionally one or more further pharmaceutically acceptable components,
wherein the or all anti-cancer agent(s) form drop particles in component f) of the composition,
wherein the particle size of at least 80 %, preferably at least 85 %, particularly preferably at least 90 % of the drop particles is ≤ 500 nm,
wherein the ratio of the total weight of component a) to the total weight of component c) is in a range of from 1:20 to 20:1, preferably of from 1:10 to 10:1, particularly preferably of from 1:8 to 7.5:1.

2. Composition according to claim 1, wherein component b) is present and
wherein the ratio of the total weight of component a) to the total weight of component b) is in a range of from 1:20 to 50:1, preferably of from 1:15 to 40:1, particularly preferably of from 1:10 to 30:1,
and/or
wherein the ratio of the total weight of component b) to the total weight of component c) is in a range of from 1:25 to 20:1, preferably of from 1:20 to 10:1, particularly preferably of from 1:10 to 5:1, especially preferably of from 1:8 to 2.5:1.

3. Composition according to claim 1 or 2, wherein component d) is present and
wherein the ratio of the total weight of component a) to the total weight of component d) is in a range of from 1:50 to 20:1, preferably of from 1:25 to 10:1, particularly preferably of from 1:20 to 6:1,
and/or
wherein the ratio of the total weight of component c) to the total weight of component d) is in a range of from 1:20 to 10:1, preferably of from 1:20 to 5:1, particularly preferably of from 1:10 to 2:1, especially preferably of from 1:5 to 1.6:1.

4. Composition according to any one of the preceding claims, wherein component e) is present and
wherein the ratio of the total weight of component a) to the total weight of component e) is in a range of from 0.5:1 to 500:1, preferably of from 1:1 to 350:1, particularly preferably of from 4:1 to 300:1,
and/or
wherein the ratio of the total weight of component c) to the total weight of component e) is in a range of from 5:1 to 150:1, preferably of from 10:1 to 100:1, particularly preferably of from 16:1 to 80:1,

5. Composition according to any one of the preceding claims, wherein the particle size of at least 30 %, preferably at least 40 %, particularly preferably at least 45 %, especially preferably 50 % of the drop particles is ≥ 250 nm and ≤ 300 nm.

6. Composition according to any one of the preceding claims, wherein the, one, two, three or more or all anti-cancer agent(s) of component a) is/are selected from the group consisting of anti-cancer agents used against a cancer type selected from the group consisting of gastrointestinal cancer, particularly gastric cancer, colorectal cancer, hepatobiliary or pancreatic cancer, appendix cancer, esophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin, preferably selected from the group consisting of cisplatin, doxorubicin, paclitaxel, oxaliplatin, clofazimine and quercetin,
and/or
wherein component b) is present and the, one, two, three or more or all immunomodulatory agent(s) of component b) is/are selected from the group consisting of curcumin, clofaximine, lutein, flavonoids like hesperidin, apigenin, hesperetin, aioene, arctigenin, β-carotene, Lycopene epigallocatechin-3-gallate, ginsan, glabridin and guinic acid,
and/or
wherein the, one, two, three or more or all surfactant(s) of component c) is/are selected from the group consisting of poloxamer, such as poloxamer 188, polysorbate, such as polysorbate 80 phosphatidylcholine, sorbitan fatty acid esters, such as span 80, vitamin E derivatives, such as D-α-Tocopheryl polyethylene glycol 1000 succinate (TPGS), polyvinyl alcohols, Macrogol glycerol ricinoleate, Polyoxyl-35-castor oil, Span, Macrogol 15 Hydroxystearate, Polyoxyl 15 Hydroxystearate and Polyethoxylated 12-hydroxystearic acid,
and/or
wherein component d) is present and the, one, two, three or more or all solvent(s) of component d) is/are selected from the group consisting of ethanol, water, acetone, ethyl acetate, chlorinated solvents, DMF, DMSO, (diluted) acetic acid, methylated solvents, tetrahydrofuran, halogenated hydrocarbons such as chloroform and dichloromethane, dioxanes, acetonitrile, polyethylene glycol, such as polyethylene glycol 400; N-methylpyrrolidone; dimethylacetamide; propylene glycol and combinations thereof,
and/or
wherein component e) is present and the, one, two, three or more or all coating material(s) of component e) is/are selected from the group consisting of celluloses, such as methyl cellulose, hydroxypropyl cellulose, sodium carboxymethyl cellulose or hydroxypropylmethyl cellulose, chitosan or carboxymethyl cellulose, polyvinyl alcohol, polyacrylic acid, polyvinylpyrrolidone, sodium alginate, pectin, carbomer, tragacanth, acacia, gelatin, starch, such as gellatinised starch, gums such as gellan gum, xanthum gum, locust bean gum or acacia gum, hydrogels, folic acid, antibodies, dextran, and serum albumin.

7. Composition according to any one of the preceding claims, wherein component e) is present and at least 50 %, preferably at least 60 %, preferably at least 70 %, preferably at least 80 %, preferably at least 90 %, preferably at least 95 % of the drop particles are coated with the or at least one, two, three or more or all of the coating material(s) of component e).

8. Method for producing a composition according to any one of claims 1 to 7, comprising or consisting of the steps
i) providing one, two, three or more anti-cancer agent(s), wherein the or all anti-cancer agent(s) is/are water-insoluble,
ii) providing one, two, three or more surfactant(s),
iii) optionally: providing one, two, three or more immunomodulatory agent(s),
iv) optionally: providing one, two, three or more solvent(s),
v) optionally: providing one, two, three or more coating material(s)
vi) optionally: providing one or more further pharmaceutically acceptable components,
vii) providing water,
viii) mixing the components provided in steps i) and ii) and optionally iii) and/or iv) and/or v) and/or vi) with the water provided in step vii), and homogenizing the composition obtained in step viii) and obtaining drop particles by applying
a) bead milling or
b) high pressure homogenization or
c) a microfluidic process or
d) combinations thereof
ix) optionally: providing one or more further pharmaceutically acceptable components and adding these to the composition obtained in step viii), wherein the addition may be performed at any time within step viii).

9. Composition according to any one of claims 1 to 7, obtainable or obtained by the method according to claim 8.

10. Lyophilisate of a composition according to any one of claims 1 to 7 and 9, obtainable or obtained by the following step
- lyophilising a composition according to any one of claims 1 to 7 and 9,
preferably wherein one, two, three or more cryoprotectant(s) is/are used,
particularly preferably wherein the, one, two, three or more or all cryoprotectant(s) is/are selected from the group consisting of trehalose, DMSO, lactose, Ethylene glycol, sorbitol, glucose, fructose, Glycerol, 2-Methyl-2,4-pentanediol (MPD), Propylene glycol, Sucrose, albumin, mannitol and combination thereof.

11. Composition according to any one of claims 1 to 7 and 9, for use in the treatment of cancer in a human subject, wherein the treatment comprises one or more administration(s) of the composition to the human subject, preferably by direct introduction into the abdomen and/or thorax.

12. Composition for use according to claim 11, wherein the treatment is directed to a cancer type selected from the group consisting of gastro-intestinal cancer, particularly gastric cancer, colorectal cancer, hepatobiliary or pancreatic cancer, appendix cancer, esophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin.

13. Composition for use according to claim 11 or 12, wherein the or one, two, three or all administration(s) of the delivery system is/are performed with an assisting tool selected from the group consisting of microneedles, spray devices, angioinjectors, or any combination thereof, preferably with a nebulizer, spraying gun or spray catheter.

14. Composition for use according to any one of claims 11 to 13, wherein the treatment is directed to prevent or delay cancer recurrence after a surgical tumour removal.

15. Composition for use according to any one of claims 11 to 14, wherein the or one, two, three or all administration(s) comprise or consist of intraperitoneal administration, intravenous injection, oral administration, intramuscular, PIPAC, or combinations thereof.
